# EUROPEAN PATENT SPECIFICATION

(11) **EP 3 897 287 B1**
(45) Date of publication and mention of the grant of the patent: **24.01.2024**
(21) Application number: 19839716.8
(22) Date of filing: 18.12.2019
(51) Int. Cl.: A45D 44/00

(54) **APPLICATOR WITH AN ACTIVE AGENT APPLIED IN A CONCENTRATION GRADIENT AND METHOD OF MAKING IT**
APPLIKATOR MIT EINEM AKTIVEN MITTEL, DER IN EINEM KONZENTRATIONSGRADIENT VERTEILT WIRD UND HERSTELLUNGSVERFAHREN DAFÜR
APPLICATEUR AVEC UN AGENT ACTIF APPLIQUÉ DANS UN GRADIENT DE CONCENTRATION ET SON PROCÉDÉ DE FABRICATION

(30) Priority: 19.12.2018 US 201862781857 P
(43) Date of publication of application: 27.10.2021
(73) Proprietor: The Procter & Gamble Company, Cincinnati, OH 45202 (US)
(72) Inventor: STANLEY, Scott Kendyl, Cincinnati, Ohio 45202 (US); RAPACH, Andrew Paul, Cincinnati, Ohio 45202 (US)
(74) Representative: P&G Patent Germany
(86) International application number: PCT/US2019/067029
(87) International publication number: WO 2020/131983

(56) References cited:
- WO-A1-99/55394
- US-A1- 2018 206 616

## Description

### FIELD OF THE DISCLOSURE

The disclosure relates to methods of making applicators having active ingredients supplied in a gradient, and more particular to applicators, such as masks, having gradients of one or more active agents in one or more regions of the applicator.

### BACKGROUND

Agents for affecting target structures are well known. Temperature affects may be induced by the application of hot or cold agents to the target. The appearance of a target may be affected by cosmetic and decorative agents. Electric current, voltages, and electric and magnetic fields may be applied to a target using local applicators. For biological targets, surface properties may be impacted by the use of topical application of moisturizers, medicaments and other treatment actives.

The effectiveness of the active agent may be impacted by the nature of the applicator available to facilitate the interaction of the active agent with the target structure and the concentration of the active agent in a target region. Typical applicators are less than precise with respect to their conformance to the target structure and the use of one-size, or a few sizes, fits all tends to compromise the actual performance of the active agent and/or make less efficient use of the concentration of the active agent. Conventional applicators typically have uniform application of actives across the mask or a patch of the mask. This can lead to over exposure of the active in areas to ensure coverage of the target zone at a desired concentration. This can also lead to abrupt ends in the presence of the active agent, which, for some actives, such as self-tanner or skin lightening products, can lead to unnatural and/or undesirable results on the skin such as harsh lines or transitions corresponding to where the active ended.

Document US 2018/0206616 A1 discloses a method for targeted application of topical agents to an isolated body part comprising the steps of capturing an image of the isolated body part, transforming the image data to mathematical model of the geometry of the isolated body part, forming an applicator mask having an applicator surface having a three dimensional shape corresponding to the isolated body part, forming a releasable membrane on the applicator surface of the applicator mask comprising one or more benefit agents disposed in one or more treatment zones of the applicator, where the adhesion of the membrane to the isolated body part is greater than the adhesion of the membrane to the applicator surface, disposing the applicator mask on the isolated body part so that the releasable membrane is in contact with the isolated body part, and removing the applicator mask from the isolated body part so that the membrane remains in contact with the isolated body part.

### SUMMARY

A method of making a treatment applicator is provided by claim 1.

A treatment applicator made by this method includes an applicator surface arranged to cover at least one target area; and an active agent applied to the applicator surface area for application to the at least one target area, wherein the active agent is applied in a concentration gradient decreasing from a first zone of the at least one target area to a second zone of the at least one target area.

A treatment applicator made by this method includes an applicator surface, a first zone of the applicator surface arranged to cover at least one target area and a second zone of the applicator surface arranged to extend over an edge or curvature adjacent the at least one target area; and an active agent applied to the applicator surface in the first and second zones, wherein the active agent is provided in a concentration gradient from the first zone to the second zone.

Gradients may be applied to a flat sheet material that will be subsequently formed into a 3D applicator, therefore, areas that will stretch to a high degree may need a higher concentration of active to maintain a desired coverage amount in the formed part.

### BRIEF DESCRIPTION OF THE DRAWINGS

While the specification concludes with claims particularly pointing out and distinctly claiming the subject matter that is regarded as the present invention, it is believed that the invention will be more fully understood from the following description taken in conjunction with the accompanying drawings. Some of the figures may have been simplified by the omission of selected elements for the purpose of more clearly showing other elements. Such omissions of elements in some figures are not necessarily indicative of the presence or absence of particular elements in any of the exemplary embodiments, except as may be explicitly delineated in the corresponding written description. None of the drawings are necessarily to scale.
Fig. 1A is a schematic illustration of an applicator fitted to a face in accordance with embodiments of the disclosure;
Fig. 1B is a schematic illustration of a face illustrating target areas for application of an active agent;
Fig. 2A is a schematic illustration of an applicator fitted to a face in accordance with embodiments of the disclosure;
Fig. 2B is a schematic illustration of an applicator fitted to a face in accordance with embodiments of the disclosure
Figure 3A is a schematic illustration of an applicator illustrating target areas for application of an active area and concentration gradients of actives within a given target area in accordance with embodiments of the disclosure;
Figure 3B is a schematic illustration of an applicator illustrating target areas for application of an active area and concentration gradients of actives within a given target area in accordance with embodiments of the disclosure;
Figure 4A is a schematic illustration of an applicator illustrating target areas for application of an active area and concentration gradients of actives within a given target area in accordance with embodiments of the disclosure in which the entire applicator is covered by one or more actives
Figure 4B is a schematic illustration of an applicator illustrating target areas for application of an active area and concentration gradients of actives within a given target area in accordance with embodiments of the disclosure in which the entire applicator is covered by one or more actives; and.

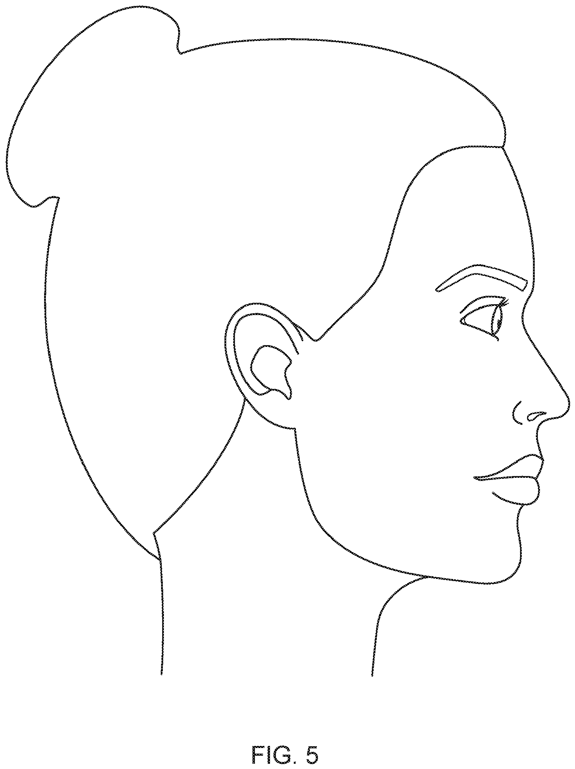

Figure 5 is a side view of a human face to which an applicator can be fitted.
Figure 6 is a process diagram illustrating a method of defining a treatment area aligned with a target area in accordance with embodiments of the disclosure;
Figure 7 is a process diagram illustrating a method of defining a treatment area aligned with a target area in accordance with embodiments of the disclosure; and
Figure 8 is a schematic illustration of an overlay of an applicator on a target area showing the treatment being defined over the target area.

### DETAILED DESCRIPTION

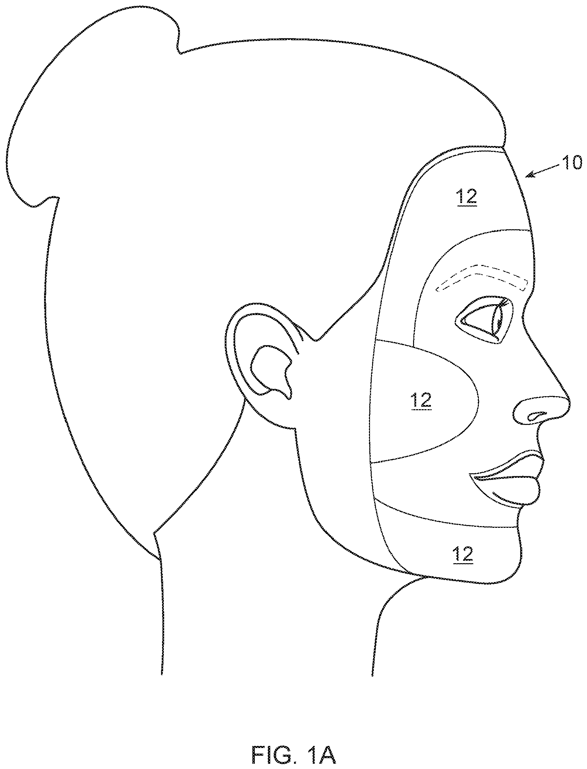

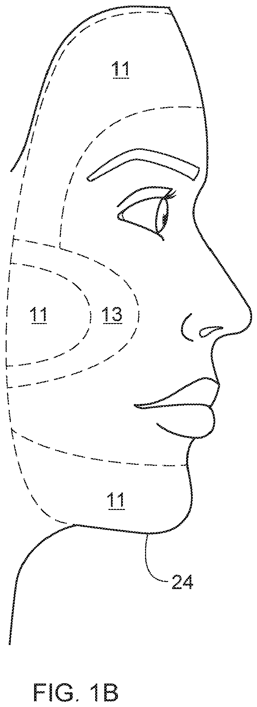

The disclosure provides a method for making an applicator, the method being according to the invention, and provides embodiments of an applicator, not according to the invention, that is the result of the method of making an applicator. An applicator for applying one or more active agents to a target surface, with at least one active agent in at least one target area 11 of the target surface being provided on the applicator in a concentration gradient. Referring to Figs. 1A and 1B, in embodiments, the applicator 10 can target one or more target areas 11 of the target surface. Target areas 11 can be adjacent another target area or can be separated from target areas 11 in an isolated region of the applicator 10. Concentration gradients of active agents can be beneficial for various active agents, for example, allowing for controlled reduction of the concentration of an active agent at the periphery of the target area 11. Concentration gradients of the active can also be beneficial in avoiding abrupt changes in the presence of the active agent. For example, in many conventional applicators, the active is provided in the target area 11 as a patch and is sized to be the same or slightly larger than the target area 11. Immediately outside this sizing there is an abrupt change in that the active agent is not present. With some active agents, for example, skin lighteners, self-tanners, and the like, this can lead to an undesirable or unnatural effect on the target surface, such as a color difference in the surface that defined by a distinct line rather than a natural blend.

A gradient change to avoid such abrupt changes in the concentration of active and associated results on the target surface can also be beneficial when the target area 11 has or is adjacent to a curvature 24. In embodiments, the concentration gradient 18 can be provided between a first zone 12 and a second zone 14 with a curvature or edge 24 zone disposed between the first and second zones or within one of the zones. Fig. 1A illustrates an applicator having a zone crossing over a curvature 24 of the target surface in the chin region. The concentration gradient 18 of the active can be a decreasing gradient from the first zone through the curvature or edge 24 zone to the second zone. The concentration gradient 18 of the active can be a decreasing gradient from the first zone 12 to the curvature or edge 24 zone, and then increasing from the curvature or edge 24 zone to the second zone. The curvature or edge 24 can be in better contact with the applicator than surrounding more planar areas. For example, a gap or bubble in the applicator can result in the surrounding areas. Reduction of the concentration of the active agent in the curvature or edge 24 zone can aid in reducing or avoiding overexposure of the curved area or edge 24 to the active, which can result from the way in which the applicator contacts the surface in this target area 11.

Applicators having actives providing in a gradient can also be advantageous when applying to a target surface that benefits from different amounts of active agent. For example, an applicator for such a target surface can have a first zone 12 in which a high concentration of active agent is beneficial and a second zone 14 in which a lower concentration of active agent is beneficial. Conventional masks typically provide the active in a single concentration, which can lead to either under exposure or over exposure of the target areas to the active in the first or second zone 14. Applicators in accordance with embodiments of the disclosure, however, can advantageously delivery the desired amount of active to each target area, without abrupt changes or gaps in the concentration.

The concentration gradients can be beneficial for blending active ingredients applied to adjacent target areas 11. For example, the target surface can have a first target area and a second target area and first and second active agents for treating the first and second target areas respectively. It can be beneficial to provide a blended area with a reduced amount of each of the active agents in the zone between the two target areas, rather than have a uniform concentration of the first active agent and the second active agent, with the agents abutting in the zone between the two target areas, rather than blending.

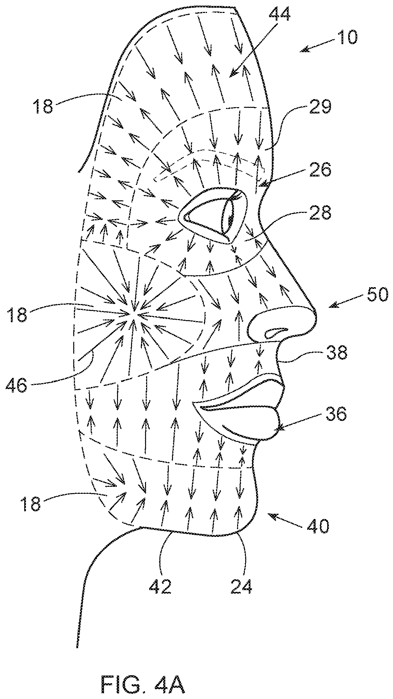

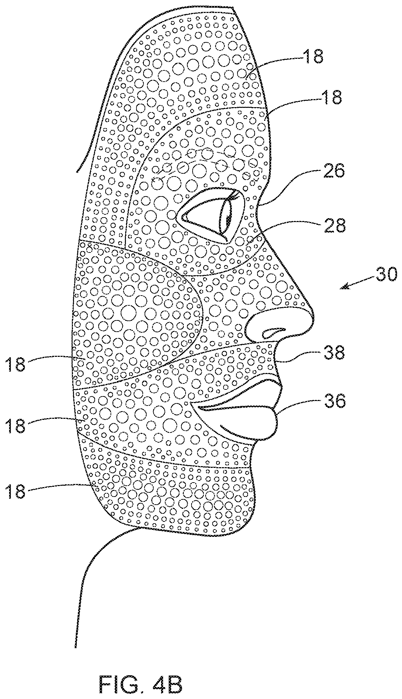

An applicator can have a surface to which one or more active agent is applied. The surface having the one or more active agents applied thereto is adapted to contact a target surface. Referring to Figs. 4A and 4B, in embodiments, the applicator can have one or more active agents applied to cover all or substantially of the surface of the applicator. Figs. 4A and 4B illustrate embodiments in which the target surface has multiple target areas and the applicator has multiple zones each having a concentration gradient 18 within the zones. The zones can have applied therein different active agents or the same active agents applied in different concentrations. For example, a first active agent can be applied in the zone surrounding the eye 26, with a concentration gradient 18 provided with increasing concentration around the lower eyelid region 28 and the upper eyelid region 29. A second active agent can be applied in a zone surrounding the mouth 36, with an increasing concentration gradient 18 being provided from the vermillion border 38 inward. A third active agent can be applied in a zone defined at the nose 30, with increasing concentration being defined at a crease in the outside edge of the nostrils. In other embodiments, the active agent in each of the zones can be the same. Additional zones can be defined, for example, in the chin region 40 and/or along the jaw line 42 to adapt the concentration for the curvature 24 in those regions. This can be useful, for example, in manufacturing to tailor the gradient for concentration differences resulting from spray coating in these regions. Other regions where concentration gradients 18 can be useful for a face mask include the cheeks 18 and the forehead 44 area. Concentration gradients 18 can also be used to target a condition. For example, on the face, acne can be particularly targeted with increased concentration of an acne medication at the site of the blemish and decreased concentration of an acne medication surrounding the blemish.

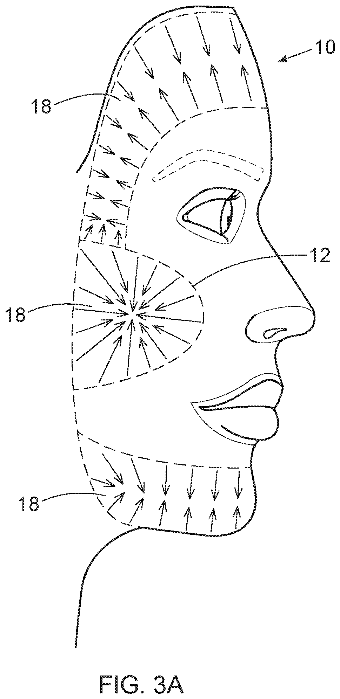

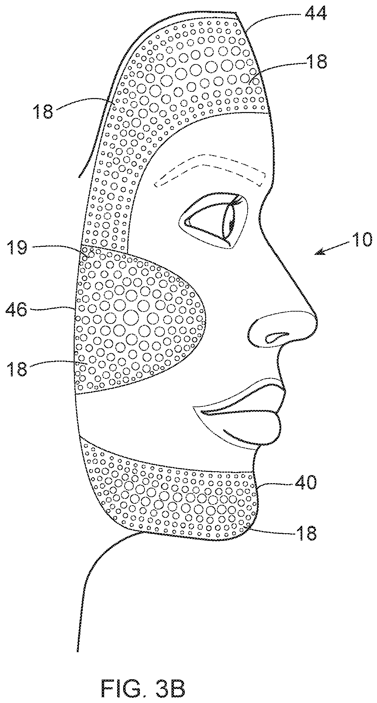

Referring to Fig. 3A and 3B, in embodiments, the applicator can have one or more active agents applied to cover one or more designated regions of the surface adapted to contact one or more target areas 11 of the target surface. Figs. 3A and 3B illustrate target areas 11 in the chin 40, forehead 44, and check 46 regions. Other regions can also be targeted. Further, as illustrated in Figs. 3A and 3B, the target areas 11 can be adjacent one another, such as the cheek region 46 and the forehead region 44 or can be separated such as the chin region 40. In embodiments, the adjacent regions, such as illustrated as cheek and forehead regions 44, 46 in Fig. 3B can include a blending region 19, where the active agents in each region are blending at appropriate concentrations using a decreasing gradient towards and in some cases within the blending region 19.

Where the applicator surface is covered or substantially covered with one or more active agents, the applicator can include one or more zone corresponding to target areas 11 in which a different amount of active agent or different active agent is to be applied. In embodiments in which the one or more active agents is applied to cover a designed region of the surface, the applicator can include in this region one or more zone corresponding to portions of the target area 11 and/or peripheral regions 13 around the target area 11 as illustrated in Fig. 1B.

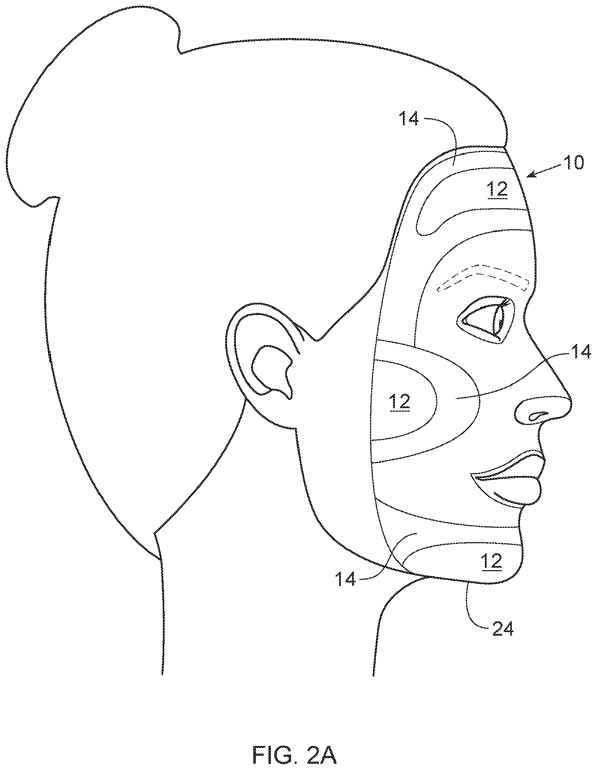

Referring to Fig 2A, for example, in embodiments, the applicator can have a first zone 12 corresponding to a portion of the target area 11 and a second zone 14 corresponding to a different portion of the target area 11. For example, the applicator can have a first zone 12 corresponding to the target area 11 and a second zone 14 corresponding to a periphery surrounding or a region adjacent to the target area 11. The target area 11 can be a peripheral area 13 of a central zone, such that an increasing gradient concentration is provided from the central zone to the outer peripheral target area 11. The peripheral area can be the first zone 12 and a central zone can be the second zone 14. The active agent can be applied to the applicator in a concentration gradient 18 decreasing from the first zone 12 to the second zone 14. Any number of zones can be provided for a given target area depending on the target area and the treatment to be applied.

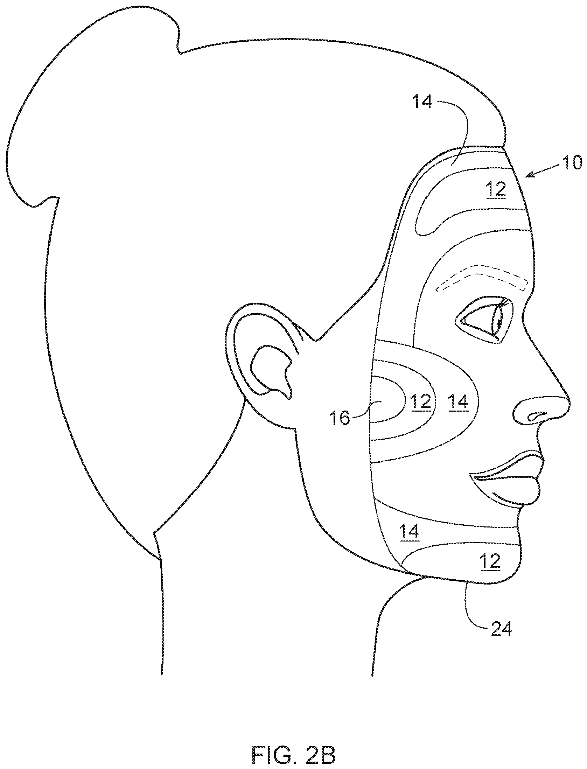

For example, a target area can have two regions in which higher concentrations of actives would be beneficial with a spacing between the two regions where reduced active concentration would be beneficial. Referring to Figure 2B, in such embodiments, the applicator can be defined to have three zones, first and third zones 12, 16 corresponding to the two regions for application of higher concentrations, and a second zone 14 interposed between the two regions corresponding to the spacing where lower concentration is beneficial. The active agent can be provided in a concentration gradient 18 decreasing from the first zone 12 to the second zone 14 and then increasing from the second zone 14 to the third zone 16.

While reference will be made herein to first and second zones 12, 14 or first, second, and third zone 12, 14, 16, and the concentration changes between such zones, it should be understood that any number of zones can be used and concentration gradient 18 can be varied between different zones. For example, the target area can be a first zone 12, a second zone 14 can be defined as a lower peripheral region of or adjacent to the target area, a third zone 16 can be defined as an upper peripheral region of or adjacent to the target area, and a fourth zone (not shown) can be defined at side peripheral regions of or adjacent to the target area. In embodiments, the applicator 10 can have a first concentration gradient 18 between the first zone 12 and the second zone 14, a second concentration gradient 18 between the first zone 12 and the third zone 16, and a third concentration gradient 18 between the first zone and the fourth zone. Further in embodiments, where zones such as the second, third and/or fourth zone are adjacent concentration gradients can be further provided between these zones. The rate of change in the various concentration gradients can be the same or different. The distance over which the concentration gradient extends between various zones can also be the same or different.

Referring to Fig. 3A, 3B, 4A, and 4B, any concentration gradient 18 can be used over a given set of zones. For example, in embodiments, the concentration gradient 18 can decrease from the first zone 12 to the second zone 14 and continually decrease through the second zone 14 until a 0 or predefined lower concentration is reached. The rate of decrease in the concentration and/or the concentration at the end of the gradient can be tailored. For example, in embodiments, a uniform concentration of an active agent can be provided on the entire surface of the applicator 10, a higher concentration of the active agent or a different active agent can be provided in a first zone 12 corresponding to a target area 11 and a concentration gradient 18 can be provided from first zone 12 to reduce the concentration of the active to the uniform concentration over a distance from the first zone 12. The distance across which the concentration gradient 18 extends can also be tailored. The concentration gradient, including the rate of change, the distance the gradient extends, and the overall change in concentration can be tailored depending on a number of factors including one or more of the active agents applied, the target area, the condition to be treated, and the size of the treatment zone.

The concentration gradient 18 can be defined through the first and second zones 12, 14 with a continual decrease in concentration from an outermost point in the first zone 12 to the opposed outermost point in the second zone 14. The concentration gradient 18 can be defined through a single zone. For example, a uniform concentration of active agent can be present in the entirety of the first zone 12 and the gradient can be provided through the second zone 14. In embodiments, the first zone 12 can correspond to the target area 11 and the second zone 14 can corresponding to a peripheral region 13 of the target area 11.

An applicator 10 can have first and third zones 12, 16 having uniform but different concentrations of active agent in the zones and a second zone 14 disposed between the first and third zone providing a gradient between two concentrations of the first and third zone 12, 16. In embodiments, different active agents can be applied in first and third zones 12, 16 and a concentration gradient 18 of each active agent can be provided in a second zone 14 disposed between the first and third zones 12, 16 to provide for blending of the active agents. Blending can be beneficial, for example, to allow for the presence of both active agents, without overexposure and/or to avoid abrupt changes in the actives that occur when two uniform concentrations of active agents abut one another.

The intended target area can be, for example, the entire face of a user or a portion of the face. In such embodiments, the applicator 10 can be a mask for covering the face or a portion or portions thereof. For example, the intended target area can be an eye area. In another or further embodiment, the target area can be the corner of the nose, outside the nostrils. Any specific region or regions of the face can be used as the target area or the entire face can be the target area.

The applicator 10 can be a two-dimensional applicator or a three-dimensional applicator. For example, the applicator 10 can be a custom-made three-dimensional face mask designed specifically for the user's face or a target portion thereof. Such custom-made three-dimensional face mask can be any of those described in U.S. Patent Application Publication Nos. 2017/008566, 2017/0354805, and 2017/0354806, the respective disclosures of which are incorporated herein by reference. For example, in embodiments, an applicator can be based on a three-dimensional scan or any other measure of a target region. For example, the applicator may be partially, substantially, or entirely shaped like a specific individual's face.

The three-dimensional applicator can include one or more registration features for improved alignment of the applicator to the target area. For example, in embodiments in which the target area is a face, a nose region or jaw-line curvature can be used as a registration feature for alignment of the mask on the face. In embodiments, where the applicator is a face mask, the mask can have custom-defined openings for one or more of the nose, mouth, and eyes. Custom-defined openings can allow for improved registration of the mask and/or coverage of treatment areas near the eyes, mouth, and nose regions.

Improved registration of the applicator with the target structure can allow for improved registration of the gradient zones with the areas of the target area to be treated. For example, a condition can be identified on a target area having a gradient of intensity. With proper registration of the applicator, an active agent can be applied in a gradient concentration mirroring the gradient of intensity of the condition. Methods of making a mask in accordance with embodiments of the disclosure can include defining at least one treatment area corresponding to a condition to be treated in the target area aligned with the target area, as detailed below.

The gradient concentration can be zoned in intensity zones larger than the gradient of intensity of the condition to ensure the proper dosage of active is applied in the high intensity regions of the condition despite some misalignment that may occur when the applicator is applied to the target area.

Conventional mask can suffer from gapping at this region, resulting in limited or no contact of various region. As a result, conventional masks can be supplied with a high concentration of the active to accommodate for poor fit. Further, they are typically supplied with uniform concentrations of active agents across the mask to accommodate for poor registration of the mask on the face to ensure active agent is applied even if the mask is out of registration.

Applicators 10 in accordance with embodiments of the disclosure can have improved registration and fit around various features, thereby allowing for the amount and location of the active agent to be highly tailored and controlled. This can advantageously allow for multiple different active agents to be used in combination on a single mask without abrupt transitions between the different actives. Applicators 10 in accordance with embodiments of the disclosure can, for example, include the blending of actives in designated regions of a target area or areas and separate application of the individual actives in other target areas.

Applicators 10 in accordance with embodiments of the disclosure can include any suitable active, cosmetic, or therapeutic agent to be applied to a target area. For example, the target area can be the face of the user. For example, active, therapeutic, and/or cosmetic agents can include active ingredients, carriers, chassis, emulsions, hydrogels, adhesives, process aides (such as thickeners, rheology modifiers, etc.). Active agents may further comprise a release layer to help active agents transfer from the applicator 10 to the target surface. Active agents may include adhesive materials, active chemical agents, absorbent materials such as absorbent gel materials or absorbent foam materials placed according to either the diagnostic scan or relative to identifiable features. As an example, it may be desirable to dispose an absorbent foam material along cheekbones, brow or nose of a scanned user's facial mask, the disposition sites may be determined according to the geometry of the representation rather than according to the diagnostic scan of the user. Active agents may be in one or more physical forms, including but not limited to: foams, liquids, powders, films, fibers, creams, gels, hydrogels, encapsulated active agents, solids, combinations of these forms and other forms. Some examples of active agents include but are not limited to: moisturizer, anti-aging, anti-wrinkle, skin tone control, anti-irritation, sensates (e.g. menthol), heating or cooling chemistries, skin tightening, hair removal, hair regrowth, fungicide, antibacterial, antiviral, surfactants, cleaning agents, copper ion eluting (such as from Cupron of Richmond, Va.), antioxidants, vitamins, sunscreen, rejuvenation agents, wound healing agents, sebum management agents, astringents, exfoliates, anti-inflammatory, leave on, overnight, dry skin, itchy skin, cracked skin, peptides, acne, scar treatments, sore muscles treatments, medicaments including pharmacological actives to treat disease states or other acute or chronic issues such as eczema, rashes, acne, cancer, cold sore, Psoriasis, Rosacea, Vitiligo, warts, Herpes, fungal infection, Actinic Keratosis, ulcers, shingles, poison ivy, and insect bites. Further, the medicaments, including pharmacological actives, can go beyond topical effect and be designed for transdermal delivery of an active into the bloodstream or other internal tissue. Examples of therapies, both prescribed and un-prescribed include: nicotine, Botox, and hormone supplements.

Exemplary active agents for cosmetic changes to the target structure include: hydrating agents, acne treating agent, anti-aging agents, ant-wrinkle agents, matte-finish compounds, under-eye hydrating agents, anti-oil agents, primer, lipstick, lip gloss, lip liner, lip plumper, lip balm, lip conditioner, lip primer, lip boosters, concealer, foundation, powder, rouge, blush, blusher, contour powder/creams, highlight, bronzer, mascara, eyeliner, and setting materials, scents, perfume or fragrance compositions (e.g. essential oils).

The inclusion of one or more scents, perfume or fragrance compositions may be applied to the applicator 10 for subsequent deposition to the face. However, a portion, or all, of the included one or more scents, perfume or fragrance compositions may act as experience agents. The experience agent provides a smell in the environs of the applicator 10 when in use. For example, the smell provided by a fragrance to suggest outdoor flower garden aroma may be desirable when applying cosmetic agents to the face of a consumer/wearer. Experiential agents need not necessarily be located on the target structure contact surface of the applicator 10. The agents may be located in a region not in contact with the target structure, such as on a non-contacting portion of the application side of the applicator 10 or anywhere on any applicator 10 side that is non-contacting to the target structure. The experience agent may be selected to accompany a selected appearance feature.

### Method of Making the Mask

A method of creating an applicator having a gradient concentration of at least one active disposed thereon includes receiving on a processor a first set of digital data representing the target area, the first set of data being received from a digital geometric representation of the target area stored on a memory or streamed in real time. The method further includes receiving on the processor a second set of digital data representing at least a portion of an applicator designed to contact the target area. The method also includes digitally overlying the second set of digital data over the first set of digital data to define digitally the contact area of the at least the portion of the applicator when applied to the target area. The method also includes defining in the digitally overlaid data a first zone of at least one target area and a second zone of the at least one target area. The defined zones are then defined as a third set of data, which is received on a processor for subsequent definition of a concentration gradient and/or generation of visual depictions of the applicator with the zones and/or the concentration gradients. The concentration gradient is defined by setting a concentration of an active agent to be applied to applicator in at least a portion of the first zone; setting a concentration of an active agent to be applied to the applicator in at least a portion of the second zone; defining a concentration gradient of the active agent between the concentration of the active agent in the first zone and the concentration of the active agent in the second zone.

The digital geometric representation of the target area, an applicator 10, and/or the manufactured three-dimensional mask can be streamed in real time, received from a memory, or received direct from the capture source, such as a three-dimensional scanner. The digital geometric representation of the target area or the application can be obtained using one or more of three-dimensional scanners, two-dimensional scanners, cameras, smartphone camera, digital applications for tablets and phones, and other known equipment for obtaining digital geometric data. An Artec Spider, available from Artec Group Palo Alto, CA is an example of a suitable three-dimensional scanner. An example mobile application for a cellular phone or table is 123D Catch from Autodesk or Bellus3D from Bellus3D.

The digital geometric representation of the target area or the applicator 10 can be used as a whole or partitioned with only a portion of the total representation being used. Furthermore, portions of the geometry derived from the scan or other imaging technique can be removed or edited from the digital geometric representation. The digital geometric representation data may be used without alteration, or the geometry of the representation may be altered. For example, digital processing (e.g. smoothing, gap filling, interpolation, down sampling, etc.) may be used to alter the digital data. For example, the digital data can be altered to be provided as a mesh to allow for measurement of various features on the digital data. For example, a two-dimensional set of data from an image or scan can be altered to provide a three-dimension representation of the two-dimensional data.

Any one or more of the various digital processing equipment, digital geometric representations, graphics programs, and graphical displays may be stored in a tangible computer readable memory or medium and/or shared or cloud-based medium, and execute one or more processors to perform the functions described herein. For example, the digital geometric representation can be obtained by a user using a smartphone camera and/or mobile application and subsequently uploaded to a manufacturer's shared memory or medium for manufacturing of the mask. In other embodiments, digital geometric representations can be obtained with scanners or other imaging devices located at the point of sale of the mask. The data from the digital geometric representations can be stored locally or on a shared medium.

The digital geometric representation of the target area or the applicator 10 can be stored on a memory or shared medium and transmitted to a processor. The digital geometric representation of the target area or the applicator 10 can be stored on a memory of the scanning or capture device and transmitted by the device or other means to a processor. For example, the scanning or capture device can store the digital geometric representation in whole or in part, including, on a buffer memory for transmitting the digital geometric representation to the processor in buffering mode.

The target area can be a human face or portion thereof and the applicator 10 can be a mask for covering the face or portion thereof. The mask can include or have applied thereto one or more cosmetic, therapeutic, and active agents for treating the face or skin or skin conditions thereon. The mask can be a two-dimensional mask (e.g. sheet mask, substrate, nonwoven, woven, knit, gel, film, foil or hydrogel mask or any other material) or a three-dimensional mask made of any material. The three-dimensional mask can be a self-supporting mask. As used herein, the term "self-supporting" means that an element of or the applicator 10 in its entirety retains a substantial portion of a defined three-dimensional shape without the aid of external support structures when resting on a horizontal surface in air. In any of the embodiments disclosed herein, the mask can be a single-dose applicator 10 or for single use having a single-dose of the active, cosmetic, and/or therapeutic. As used herein, the term single-dose means an applicator 10 comprising sufficient active agents to afford a user only a single application of the active agent via the applicator 10. The mask can be for multiple use. For example, active, cosmetic, and/or therapeutic agents can be applied and successively reapplied for multi-use. In any of the embodiments disclosed herein, the mask can be disposable. As used herein, the term disposable refers to applicators 10 intended to be discarded after use rather than durable or semi-durable implements intended for multiple uses either with or without the reapplication of an active agent. In any embodiment, the mask can be a durable item suitable for washing by hand or in a dishwasher or clothing washing machine.

The target area may be a human face. The digital representation of the human face can be constrained in space from the backside to represent the bone internal to the skull. For example, the inner surface of the digital representation, corresponding to the underlying bone is treated as a rigid surface and constrained in space. The nodes on the inner surface of the skin are thus fixed in place and not allowed to move. This can be accomplished in known finite element simulation packages, for example, as a boundary condition.

The digital representation can be further modified to have mechanical properties simulating that of the target area. For example, when the target area is a human face the digital representation or resulting mesh can be given a thickness representing the skin surface. For example, the thickness can be about 0.5 mm to about 4mm, about 1 mm to about 3 mm, or about 2 mm to about 4 mm. Other suitable thickness can be about 0.5, 1, 2, 3, or 4, mm. The thickness can be constant. In embodiments, the thickness can vary according to different regions of the target area or entire face. The digital representation or set of data resulting from the digital representation can be given material surface properties as well. For example, where the target area is the human face, the digital representation or digital data can be given properties to simulate the mechanical properties of the dermis and epidermis layers. For example, a stiffness model can be used to simulate the mechanical properties for the epidermis and dermis as a single bulk layer. The model can include specification of one or more properties including for example, the stiffness, Poisson ratio, and viscoelastic behaviors. Alternately, the mechanical properties of the dermis and epidermis layers can be simulated as two separate layers.

Any of the foregoing properties or materials can be simulated in the second set of data where such materials or ingredients may affect the mechanical properties of the applicator 10, the positioning and/or retention of the applicator 10 on the target surface.

Overlaying of the applicator 10 and a target area can include converting the first set of data to a first mesh and converting the second set of data corresponding to the applicator 10 to a second mesh and aligning the two meshes using software such as, but not limited to, Artec Studio 12 Professional (Artec Software). Any of the disclosure herein is similarly applicable to the overlying of the third and first sets of data or any other set of data associated with an applicator 10 and target area. The meshes can be imported into the software in any suitable file format, including, for example, STL, OBJ, PLY, and other 3D mesh file formats. Once imported, the first mesh and the second mesh are manually brought into rough alignment. The first mesh, corresponding to the target surface, can be designated as fixed or registered and the second mesh can be designated as unregistered, thereby allowing the second mesh to be moved relative to the first mesh. Once roughly aligned, an align feature of the software can be used to bring the first and second meshes into refined alignment.

The first and second set of data can be overlaid to simulate application of the applicator 10 to the target surface and a digital representation of the overly can be generated. For example, the second set of data corresponding to the applicator 10 can be positioned a distance from the first set of data and a digitally applied force can be used to push the second set of data against the first set of data, thereby simulating application of the applicator 10 to the target area. For example, the applied force can be a distributed force on the applicator 10 or can be a localized force on the applicator 10. For example, an initial load can be provided as localized points of force, can be applied to push the second set of data to the first set of data, simulating application of the applicator 10 and positioning of the user's fingers on the applicator 10 applying the localized load to position the applicator 10 onto the target area. Optionally, a distributed force can be applied to the second set of data after it is pushed against the first set of data, simulating a user applying further force across the applicator 10 to better adhere or smooth the applicator 10 to the target area. The adhesive force can also be used or incorporated into the force simulation for application and retention of applicator 10 on the target surface. For example, wet applicators 10 can include agents such as lotions, therapeutic agents, and other cosmetic agents that provide adhesive force. Additionally, or alternatively, wet applicators 10 can include a water component or have water added thereto to make the applicator 10 adhere to the target area. Adhesive force of an applicator 10 can be assessed using a peel test, in which the force required to peel the wet applicator 10 away from the target area 11 is measured, and provides the resulting adhesive force per unit area. Such force can be incorporated into the set of data representing the applicator 10 to incorporate the adhesive force as an element to the simulation of the applicator 10 being applied to and retained on the target area 11. Any suitable applications of force and associated loads can be used and will vary depending on the target area 11 and type of applicator 10 being applied to the target area 11. Suitable force profiles and loads can be readily determined by the skilled person based on typical application of the applicator 10 or measured by actual application of an applicator 10.

The applicator 10 can be a two-dimensional applicator 10. A finite element analysis can be used to bring the flat applicator 10 surface into contact with a three-dimensional target area 11 before application of the load.

The method can include a further step of removing the load after the second set of data is pushed into contact with the first set of data. The load can be removed for example, when a force balance is detected and steady state of force is achieved. The overlaid data can be manipulated in embodiments to include a representation of the cohesive force of the applicator 10 resulting, for example, from the ingredients applied to the inner surface of the applicator 10.

A static load or dynamic load can be used. Application of a dynamic load can be applied to allow the data sets to move relative to each other. Steady state can be detected by a balance of force, and representing the point at which the objects stop moving relative to each other. The load can then be removed. The data sets representing the applicator 10 and the target area 11 can again be allowed to move relative to each other until motion ceases. For example, this can simulate any compression of the skin layer and then subsequent relaxation and motion after force is removed.

Once the overlaid model reaches a converged solution one or more zones of the contact area can be defined. In embodiments, the first set of data can include identification of a condition to be treated in a target area 11, including information such as the type of condition, the intensity of the condition, and the location of the condition. For example, the first set of data can include identification of the location of the condition and an intensity map of the condition. In embodiments, the method can include defining one or more zones on the overlaid model representing the applicator 10 corresponding to the condition. For example, a first zone 12 can be defined surrounding the most intense region of the condition requiring the highest concentration of active agent, a second zone 14 can be defined at a lower intensity region of the condition requiring a lower concentration of the active agent. The method can further include generating a third set of data corresponding to the zones defined on the applicator 10 from the overlay model. The method can include in embodiments, generating a visual representation of the third set of data illustrating an applicator and the zones defined thereon.

The method further includes defining a concentration gradient 18 between the first and second zones 12, 14. The condition may have a gradient of intensity and a gradient of active agent concentration can be defined corresponding to the gradient of intensity of the condition. In embodiments, the third set of data can be modified to include representation of the concentration gradient 18. The method can include generating a visual representation of the third set of data illustrating an applicator, the zones defined therein, and the concentration gradients in the zones. In alternative or further embodiments, the concentration gradient 18 can be programmed into equipment for application of the active agent to the applicator 10 after the applicator 10 is created. Any of the foregoing described zoning and/or concentration gradient 18 can be used in the methods of the disclosure.

The method can include defining at least one treatment area for containing at least a portion of the concentration gradient 18, with the at least one treatment area being aligned with the target area. The concentration gradient 18 of the active agent is entirely contained within the at least one treatment area. The at least one treatment area may contain only a portion of the concentration gradient 18. For example, a first zone of the gradient can lie within the at least one treatment area and a second zone of the gradient can be defined at the periphery of the at least one treatment area. The method described herein can be used to define at least one treatment area and a separate method can be used for defining a gradient concentration.

The method of defining at least one treatment area can include determining a location and/or registration of a treatment area 12 of an applicator 10 having an active agent with a condition present on a target area 11 to be treated with the active agent. The method can include defining a treatment area 12 on an applicator 10 having a location and size corresponding to a location and size of a condition present on a target area 11 to be treated with an active agent applied to the applicator 10 in the treatment area 12. The method can include defining the treatment area 12 to accommodate for potential misalignment of the applicator 10 when applied to the target area 11 to ensure that the condition is contacted with the active agent present in the treatment area. The method can include creating an applicator 10 having a treatment area 12 registered with a condition present on a target area 11 to be treated with the active agent. Throughout the disclosure, the term "active agent" will be used and should be understood to include therapeutic, cosmetic, medicinal, and other actives for treating or applying to a condition.

Methods in accordance with embodiments can beneficially allow targeted location of a treatment area 12 to provide efficient contact by an active agent of a condition in a treatment area. This can allow active agents to be more localized in treating a condition and/or allow multiple active agents to be utilized on a single applicator 10 for treating different conditions on the target area 11 with localized treatment of each condition. In embodiments, the applicators 10 can be custom-made applicators 10 that have significantly improved registration to a target area 11 as compared to one-size fits all applicators 10. Such applicators 10 can allow for smaller treatment areas 12 to be defined, as less tolerance for shifting or misalignment of the applicator 10 on the target area 11 needs to be accounted for in such custom-made applicators 10. Such custom-made three-dimensional applicators 10 can be any of those described in U.S. Patent Application Publication Nos. 2017/008566, 2017/0354805, and 2017/0354806.

The applicator 10 can be a two-dimensional structure, or a three-dimensional structure. The methods in accordance with embodiments of the disclosure can illustrate the improvement in registration of a treatment area 12 with a condition to be treated on the target area 11

Throughout the discussion below reference will be made to the second set of data. It should be understood herein that similar steps, adjustments, manipulations, and uses of the data disclosed herein with reference to the second set of data can be applicable to the third set of data or fourth set of data or any set of data similarly associated with an applicator.

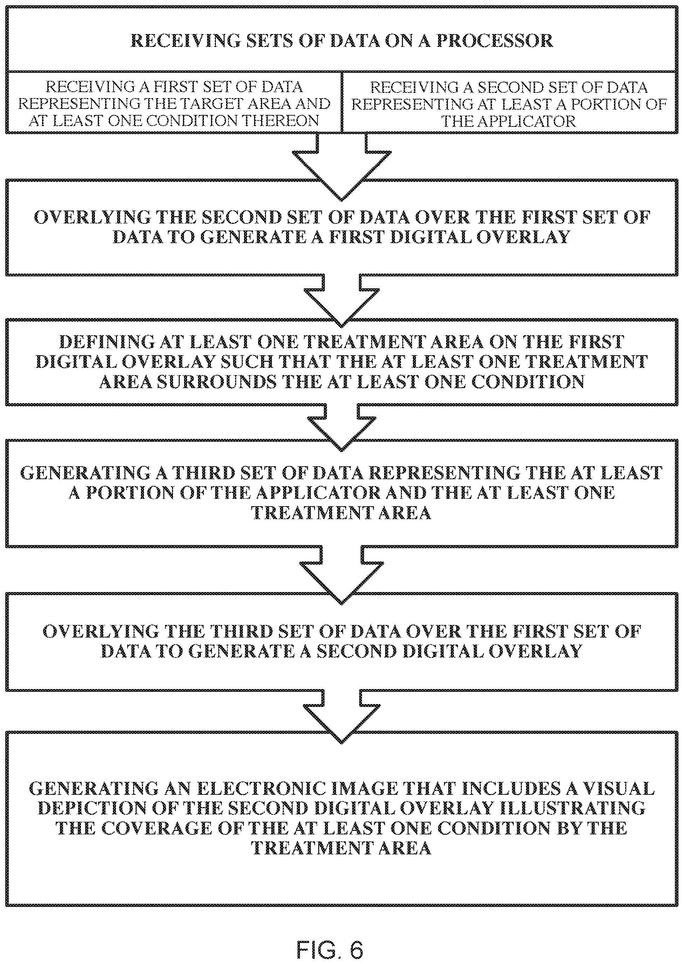

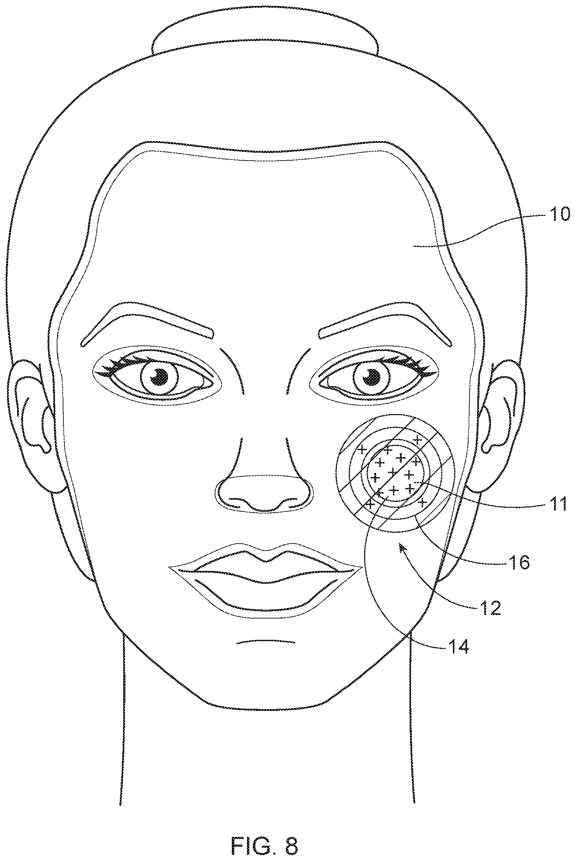

Referring to Fig. 6, a method of registering an active agent with a condition to be treated by the active agent and/or defining a treatment area 12 on the applicator 10 can include receiving on a processor a first set of data representing the target area 11 and at least one condition disposed on the target area 11 to be treated by the active agent. The method can further includes receiving on a processor a second set of data representing at least a portion of an applicator 10 designed to contact the target area 11. The method can further include digitally overlying the second set of data over the first set of data to generate a first digital overlay. The method can also include digitally defining at least one treatment area 12 on the first digital overlay such that the at least one treatment area 12 surrounds the at least one condition. The method can also include generating a third set of data representing the at least a portion of the applicator 10 and the at least one treatment area. The method can include digitally overlaying the third set of data over the first set of data to generate a second digital overlay representing coverage of the at least one condition by the at least one treatment area. Referring to Fig. 8, the method can also include generating a first electronic image that visually depicts the second digital overlay, showing the coverage of the at least one condition by the at least one treatment area.

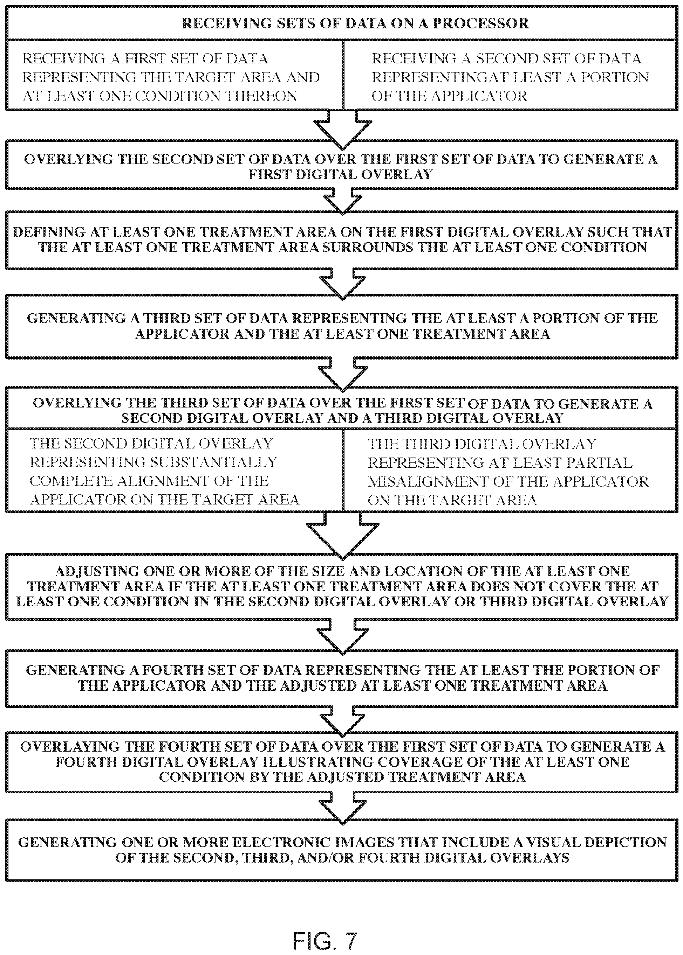

Referring to Fig. 7, the method can include definition of the treatment area 12 to accommodate misalignment of the applicator 10 when applied. In such embodiments, the method can include receiving on a processor a first set of data representing the target area 11 and at least one condition disposed on the target area 11 to be treated by the active agent. The method further includes receiving on a processor a second set of data representing at least a portion of an applicator 10 designed to contact the target area 11. The method can further include digitally overlying the second set of data over the first set of data to generate a first digital overlay. The method can also include digitally defining at least one treatment area 12 on the first digital overlay such that the at least one treatment area 12 surrounds the at least one condition. The method can also include generating a third set of data representing the at least a portion of the applicator 10 and the at least one treatment area. The method also includes digitally overlaying the third set of data over the first set of data to generate a second digital overlay. In the second digital overlay, the applicator 10 can be digitally aligned with the target area 11 to have perfect or near perfect alignment between the target area 11 and the applicator. The method can further include digitally overlaying the third set of data over the first set of data to generate a third digital overlay representing at least some misalignment between the applicator 10 and the target area 11. For example, for a custom 3D mask, the target chemistry can be delivered to the target location on the target surface with good accuracy, hitting the target within a 2 mm +/- 1 mm average offset whereas with a 2D substrate mask applicator, the offset can be as much as 6 mm on average +/- 3 mm. So, misalignments can be quite large.

The treatment area on the applicator can totally or substantially cover the target area, the treatment area on the applicator can cover 99% or the target area, 95%, 90%, 80%, 75%.

The user can select regions of interest for treatment or they can be selected automatically. The strength of actives (thickness, amount, or percentage of active ingredient) can also be changed per region automatically or selected by the user. For example, Nordson picojet systems can be used to deposit chemistry in lines or dots onto a 2D or 3D surface. The digital overlaying of the third set of data and the first set of data can be repeated with various misalignments of the applicator 10 and the target area 11. The misalignments can be different in the degree of misalignment and/or the direction of misalignment. This can be beneficial in capturing misalignments that can occur when an applicator 10 is actually applied to a target area 11 by a user.

Where multiple overlays are generated to represent misalignment of the applicator 10 and the target area 11, the at least one treatment area 12 can be analyzed in each overlay and adjusted if needed to maintain coverage of the condition. For example, the size and/or location of the at least one treatment area 12 can be adjusted if needed to accommodate a misalignment. The method can then further include generate a fourth set of data representing the at least the portion of the applicator 10 and the adjusted at least one treatment area. This fourth set of data can be digitally overlaid with the first set of data to generate a fourth digital overlay. While reference is made herein to a fourth digital overlay, it should be understood that numbering of the overlay could be different if multiple misalignment overlays are generated by overlay of the third set of data with the first set of data.

The method can further include generating one or more electronic images that each includes a visual depiction of any one or more of the digital overlays. For example, in an embodiment, the method can include generating an electronic image of second digital overlay (showing alignment), third digital overlay (showing misalignment), and fourth digital overlay (showing the adjusted treatment area) to illustrate how the treatment area 12 was effectively adjusted to allow for coverage of the condition despite misalignment. An electronic image of only the final digital overlay showing the final applicator 10 and defined treatment area 12 overlaid over the target area 11 and condition. Any number of electronic images and comparisons there-between can be generated in embodiments of the disclosure. Any known methods or software for generating an electronic image providing a visual depiction can be used.

The method can include generating a visual depiction comparing the first electronic image to the second or third (and/or any subsequent) electronic image. For example, the visual depiction can be a side-by-side view of the first and second electronic images. The method can include displaying the first electronic image before the second electronic image. The method can include displaying the second electronic image before the first electronic image. Any know methods or software for generating a visual depiction of one or more sets of data or electronic images can be used.

Any number of conditions and associated treatment areas 12 can be present. A condition can be divided into two or more treatment areas 12. Two or more conditions can be combined to be covered by a singed defined treatment area. The target area 11 can include conditions that are dispersed in isolated regions across the target area 11 and/or conditions that are adjacent other conditions. One or more active agents can be used for treating various conditions. The method can include defining a treatment area 12 for each condition in the treatment area.

The electronic image can provide a visual depiction that includes illustration of the concentration gradient in the treatment area.

The digital geometric representation of the target area 11, and/or an applicator 10 can be streamed in real time, received from a memory, or received direct from the capture source, such as a three-dimensional scanner. The digital geometric representation of the target area 11 or the applicator 10 can be obtained using one or more of three-dimensional scanners, two-dimensional scanners, cameras, smartphone camera, digital applications for tablets and phones, and other known equipment for obtaining digital geometric data. An Artec Spider, available from Artec Group Palo Alto, CA is an example of a suitable three-dimensional scanner. An example mobile application for a cellular phone or table is 123D Catch from Autodesk or Bellus3D from Bellus3D.

The first set of data can be received from a digital geometric representation of the target area 11 stored on a memory or streamed in real time. The digital geometric representation can include further information regarding the target area 11, such as coloring, texture, and other relevant information regarding the target area 11 and the condition contained therein. For example, the first set of data can include information regarding intensity of the condition. Such information can be obtained through fluorescence imaging, heat imaging, Visia scanners, Vectra scanners or any instruments that use various types of light, polarized light, certain wavelengths of electromagnetic radiation to measure or diagnose skin condition. The first set of data can be modified to include information regarding the condition which may not be captured by the digital geometric representation.

The target area 11 is a human face. The digital representation of the human face can be constrained in space from the backside to represent the bone internal to the skull. For example, in embodiments, the inner surface of the digital representation, corresponding to the underlying bone is treated as a rigid surface and constrained in space. The nodes on the inner surface of the skin are thus fixed in place and not allowed to move. This can be accomplished in known finite element simulation packages, for example, as a boundary condition.

The digital representation can be further modified to have mechanical properties simulating that of the target area 11. For example, when the target area 11 is a human face the digital representation or resulting mesh can be given a thickness representing the skin surface. For example, the thickness can be about 0.5 mm to about 4mm, about 1 mm to about 3 mm, or about 2 mm to about 4 mm. Other suitable thickness can be about 0.5, 1, 2, 3, or 4, mm. The thickness can be constant. In embodiments, the thickness can vary according to different regions of the target area 11 or entire face. The digital representation or set of data resulting from the digital representation can be given material surface properties as, well. For example, where the target area 11 is the human face, the digital representation or digital data can be given properties to simulate the mechanical properties of the dermis and epidermis layers. For example, a stiffness model can be used to simulate the mechanical properties for the epidermis and dermis as a single bulk layer. The model can include specification of one or more properties including for example, the stiffness, Poisson ratio, and viscoelastic behaviors. Alternately, the mechanical properties of the dermis and epidermis layers can be simulated as two separate layers.

The second set of data represents the applicator. The second set of data can be manipulated, for example, from a digital representation to a mesh. The second set of data can be further manipulated to include mechanical properties of the material of the applicator. For example, the applicator 10 can be rigid and such rigidity can be simulated in the second set of data. In other embodiments, the applicator 10 can be flexible. In embodiments in which the second set of data represents a two-dimensional mask, mechanical properties of a wet cotton substrate SKII FTE mask can be used, for example.

The overlaying of the data representing the applicator 10 and the data representing the target area 11 can include converting the first set of data to a first mesh and converting the second set of data corresponding to the applicator 10 to a second mesh and aligning the two meshes using software such as, but not limited to, Artec Studio 12 Professional (Artec Software). Any of the disclosure herein is similarly applicable to the overlying of the third and first sets of data or any other set of data associated with an applicator 10and target area 11. The meshes can be imported into the software in any suitable file format, including, for example, STL, OBJ, PLY, and other 3D mesh file formats. Once imported, the first mesh and the second mesh are manually brought into rough alignment. The first mesh, corresponding to the target surface, can be designated as fixed or registered and the second mesh can be designated as unregistered, thereby allowing the second mesh to be moved relative to the first mesh. Once roughly aligned, an align feature of the software can be used to bring the first and second meshes into refined alignment. Once aligned, both meshes can be selected and a measurement feature of the software can be used to calculate a distance between the two meshes at one or more points. For example, a surface-distance map calculation can be used. In embodiments, the search distance can be selected to be up to 10 mm, which represents the maximum distance between points in space the program will search for calculating separation distance. If a gap is greater than the search distance, that gap will not be included in the calculation. Any suitable search distance can be used. Once calculated, an electronic image illustrating the calculated distances graphically can be displayed.

The first and second set of data can be overlaid to simulate application of the applicator 10 to the target surface. Any of the disclosure herein is similarly applicable to the overlying of the third and first sets of data or fourth and first sets of data or any other sets of data representing the applicator 10 to overlay onto the first set of data. For example, the second set of data corresponding to the applicator 10 can be positioned a distance from the first set of data and a digitally applied force can be used to push the second set of data against the first set of data, thereby simulating application of the applicator 10 to the target area 11. For example, the applied force can be a distributed force on the applicator 10 or can be a localized force on the applicator. For example, in embodiments an initial load can be provided as localized points of force, can be applied to push the second set of data to the first set of data, simulating application of the applicator 10and positioning of the user's fingers on the applicator 10 applying the localized load to position the applicator 10 onto the target area 11. Optionally, a distributed force can be applied to the second set of data after it is pushed against the first set of data, simulating a user applying further force across the applicator 10 to better adhere or smooth the applicator 10 to the target area 11. The adhesive force can also be used or incorporated into the force simulation for application and retention of applicator 10 on the target surface. For example, wet applicators 10 can include agents such as lotions, therapeutic agents, and other cosmetic agents that provide adhesive force. Additionally, or alternatively, wet applicators 10 can include a water component or have water added thereto to make the applicator 10 adhere to the target area 11. The adhesive force of an applicator 10 can be assessed using a peel test, in which the force required to peel the wet applicator 10 away from the target area 11 is measured, and provides the resulting adhesive force per unit area. Such force can be incorporated into the set of data representing the applicator 10 to incorporate the adhesive force as an element to the simulation of the applicator 10 being applied to and retained on the target area 11. Any suitable applications of force and associated loads can be used and will vary depending on the target area 11 and type of applicator 10 being applied to the target area 11. Suitable force profiles and loads can be readily determined by the skilled person based on typical application of the applicator 10or measured by actual application of an applicator.

The applicator 10 can be a two-dimensional applicator. A finite element analysis can be used to bring the flat applicator 10 surface into contact with a three-dimensional target area 11 before application of the load.

The method can include a further step of removing the load after the second set of data is pushed into contact with the first set of data. The load can be removed for example, when a force balance is detected and steady state of force is achieved. The overlaid data can be manipulated in embodiments to include a representation of the cohesive force of the applicator 10 resulting, for example, from the ingredients applied to the inner surface of the applicator.

A static load or dynamic load can be used. Application of a dynamic load can be applied to allow the data sets to move relative to each other. Steady state can be detected by a balance of force, and representing the point at which the objects stop moving relative to each other. The load can then be removed. The data sets representing the applicator 10and the target area 11 can again be allowed to move relative to each other until motion ceases. For example, this can simulate any compression of the skin layer and then subsequent relaxation and motion after force is removed.

Once the overlaid model reaches a converged solution the at least one treatment area 12 can be defined or adjusted by defining a perimeter of the target area 11and defining an expanded perimeter that surrounds the target area 11 perimeter and is spaced outwardly a distance from at least one point perpendicular from the treatment area perimeter. The expanded perimeter defining the at least one treatment area 12 is spaced outwardly perpendicular from all points of the perimeter of the target area 11. The expanded perimeter is spaced outwardly perpendicular from at least one point of the target area 11 perimeter by about 2 mm.

As discussed above, the overlaid model can be programmed to reach a converged solution having a misalignment between the set of data representing the applicator 10 and the set of data representing the target area 11 to simulate actual misalignment that can occur when the applicator 10 is applied by the user. In general, misalignment can be most significant for two-dimensional applicators where a user seeks to first align the eye holes and other portions may be out of alignment due to rotational offsets or lateral offsets. Another source of misalignment comes from two dimensional substrates deforming onto a three-dimensional target surface, which will inherently not match with perfect alignment the curvature of the three-dimensional target area. Such misalignment can be incorporated into the model when overlying a two-dimensional substrate over the first set of data representing the target area 11.

Misalignment can be accommodated by expanding the size of the at least one treatment area. For example, in embodiments, the outward spacing between the target area 11 perimeter and the expanded perimeter defining the at least one treatment area 12 can be selected to accommodate a misalignment, ensuring that even if misaligned the target area 11 will be contacted by the at least one treatment area. For example, the size of the treatment area can be expanded in one or more directions or can be expanded in all directions from the perimeter of the target area 11.

Indicia may be present on the applicator or otherwise, to indicate the gradient(s) and the appropriate application direction for a user to achieve the desired effect. For example, indicia may be provided on the surface of the applicator to which the active agent is applied. In other embodiments, indicia may be provided on the outer surface of the applicator opposite the surface to which the active agent is applied

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

## Claims

1. A method of making a treatment applicator (10) for human skin, comprising:
receiving on a processor a first set of digital data representing the target area (11), the first set of data being received from a digital geometric representation of the target area (11) stored on a memory or streamed in real time;
receiving on the processor a second set of digital data representing at least a portion of an applicator (10) designed to contact the target area (11);
digitally overlying the second set of digital data over the first set of digital data to define digitally the contact area of the at least the portion of the applicator (10) when applied to the target area (11),
defining in the digitally overlaid data a first zone (12) of the target area (11) and a second zone (14) of the target area (11),
receiving on the processor a third set of digital data representing the at least the portion of the applicator (10) designed to contact the target area (11) having the first and second zones (12, 14) digitally defined thereon;
setting a concentration of an active agent suitable for treating human skin to be applied to the applicator (10) in the first zone (12);
setting a concentration of an active agent suitable for treating human skin to be applied to the applicator (10) in the second zone (14);
defining a concentration gradient of the active agent between the concentration of the active agent in the first zone (12) and the concentration of the active agent in the second zone (14), wherein the concentration gradient is decreasing from the first zone (12) to the second zone (14); and
creating the applicator (10) having the concentration gradient of the active agent.

2. The method of claim 1, further comprising generating an electronic image that includes a visual depiction of the concentration gradient when the applicator (10) is applied to the target area (11).

## Patentansprüche

1. Verfahren zum Herstellen eines Behandlungsapplikators (10) für menschliche Haut, umfassend:
Empfangen, auf einem Prozessor, eines ersten Satzes von digitalen Daten, der den Zielbereich (11) darstellt, wobei der erste Datensatz von einer digitalen geometrischen Darstellung des Zielbereichs (11) empfangen wird, die auf einem Speicher gespeichert ist oder in Echtzeit gestreamt wird;
Empfangen, auf dem Prozessor, eines zweiten Satzes von digitalen Daten, der wenigstens einen Teil eines Applikators (10) darstellt, der ausgebildet ist, um den Zielbereich (11) zu berühren;
digitales Überlagern des zweiten Satzes von digitalen Daten über den ersten Satz von digitalen Daten, um den Berührungsbereich des wenigstens des Teils des Applikators (10) digital zu definieren, wenn er auf den Zielbereich (11) aufgebracht wird,
Definieren, in den digital überlagerten Daten, einer ersten Zone (12) des Zielbereichs (11) und einer zweiten Zone (14) des Zielbereichs (11),
Empfangen, auf dem Prozessor, eines dritten Satzes von digitalen Daten, die den wenigstens den Teil des Applikators (10) darstellen, der ausgebildet ist, um den Zielbereich (11), der die erste und die zweite Zone (12, 14) darauf digital definiert aufweist, zu berühren;
Einstellen einer Konzentration eines Wirkstoffs, der zum Behandeln von menschlicher Haut geeignet ist, der auf den Applikator (10) in der ersten Zone (12) aufgebracht werden soll;
Einstellen einer Konzentration eines Wirkstoffs, der zum Behandeln von menschlicher Haut geeignet ist, der auf den Applikator (10) in der zweiten Zone (14) aufgebracht werden soll;
Definieren eines Konzentrationsgradienten des Wirkstoffs zwischen der Konzentration des Wirkstoffs in der ersten Zone (12) und der Konzentration des Wirkstoffs in der zweiten Zone (14), wobei der Konzentrationsgradient von der ersten Zone (12) zu der zweiten Zone (14) abnimmt; und
Erstellen des Applikators (10), der den Konzentrationsgradienten des Wirkstoffs aufweist.

2. Verfahren nach Anspruch 1, ferner umfassend ein Erzeugen eines elektronischen Bildes, das eine visuelle Abbildung des Konzentrationsgradienten einschließt, wenn der Applikator (10) auf den Zielbereich (11) aufgebracht wird.

## Revendications

1. Procédé de fabrication d'un applicateur de traitement (10) pour une peau humaine, comprenant :
la réception sur un processeur d'un premier ensemble de données numériques représentant la zone cible (11), le premier ensemble de données étant reçu à partir d'une représentation géométrique numérique de la zone cible (11) stockée sur une mémoire ou diffusée en temps réel ;
la réception sur le processeur d'un deuxième ensemble de données numériques représentant au moins une partie d'un applicateur (10) conçu pour entrer en contact avec la zone cible (11) ;
la superposition numérique du deuxième ensemble de données numériques sur le premier ensemble de données numériques pour définir numériquement la zone de contact de l'au moins la partie de l'applicateur (10) lorsqu'elle est appliquée à la zone cible (11),
la définition dans les données superposées numériquement d'une première zone (12) de la zone cible (11) et d'une seconde zone (14) de la zone cible (11),
la réception sur le processeur d'un troisième ensemble de données numériques représentant l'au moins la partie de l'applicateur (10) conçue pour entrer en contact avec la zone cible (11) ayant les première et seconde zones (12, 14) définies numériquement sur celle-ci ;
le réglage d'une concentration d'un agent actif adapté pour traiter une peau humaine à appliquer à l'applicateur (10) dans la première zone (12) ;
le réglage d'une concentration d'un agent actif adapté pour traiter une peau humaine à appliquer à l'applicateur (10) dans la seconde zone (14) ;
la définition d'un gradient de concentration de l'agent actif entre la concentration de l'agent actif dans la première zone (12) et la concentration de
l'agent actif dans la seconde zone (14), dans lequel le gradient de concentration diminue de la première zone (12) à la seconde zone (14) ; et
la création de l'applicateur (10) ayant le gradient de concentration de l'agent actif.

2. Procédé selon la revendication 1, comprenant en outre la génération d'une image électronique qui comporte une représentation visuelle du gradient de concentration lorsque l'applicateur (10) est appliqué à la zone cible (11).
